## Europäisches Patentamt

## European Patent Office

## Office européen des brevets

(19)

(11) Veröffentlichungsnummer: **0 095 698**
**B1**

(12) **EUROPÄISCHE PATENTSCHRIFT**

(45) Veröffentlichungstag der Patentschrift:
**04.09.85**

(21) Anmeldenummer: **83105098.4**

(22) Anmeldetag: **24.05.83**

(51) Int. Cl.⁴: **C 07 C 63/30**, C 07 C 63/24,
C 07 C 51/58

(54) Verfahren zur Herstellung von Terephthalsäure- und Isophthalsäuredichlorid.

(30) Priorität: **02.06.82 DE 3220729**

(43) Veröffentlichungstag der Anmeldung:
**07.12.83 Patentblatt 83/49**

(45) Bekanntmachung des Hinweises auf die Patenterteilung:
**04.09.85 Patentblatt 85/36**

(84) Benannte Vertragsstaaten:
**BE DE FR GB IT NL**

(56) Entgegenhaltungen:
**DE - B - 2 158 551**

(73) Patentinhaber: **BAYER AG, Konzernverwaltung RP
Patentabteilung, D-5090 Leverkusen 1 Bayerwerk (DE)**

(72) Erfinder: **Böckmann, Walter, Dr., Fabritiusstrasse 19,
D-4150 Krefeld 11 (DE)**
Erfinder: **Brühne, Friedrich, Dr., Fabritiusstrasse 24,
D-4150 Krefeld 11 (DE)**
Erfinder: **Lipper, Karl-August, Dr., Deswatinesstrasse 14,
D-4150 Krefeld 1 (DE)**

ACTORUM AG

## Beschreibung

Die Erfindung betrifft ein verbessertes Verfahren zur Herstellung von Terephthalsäure- und Isophthalsäuredichlorid durch Chlorieren von Terephthalsäure- und Isophthalsäuredimethylester.

Die Herstellung aromatischer Mono- und Polycarbonsäurechloride durch Chlorierung der entsprechenden Methylester bei erhöhten Temperaturen und gegebenenfalls in Gegenwart von Licht, oder die Seitenkettenchlorierung aromatischer Verbindungen fördernder Katalysatoren ist bekannt. So ist in der Deutschen Patentschrift Nr. 1064495 ein Verfahren zur Herstellung von Chloriden aromatischer Mono- und Polycarbonsäuren beschrieben, gemäss dem die entsprechenden Methylester mit Chlor in Gegenwart von Licht bei Temperaturen von 100 bis 220° C, vorzugsweise 150 bis 180° C, chloriert werden. Gemäss US-Patentschrift Nr. 2865959 werden aromatische Säurechloride durch Einwirkung von Chlorgas auf Alkylester aromatischer Carbonsäuren bei Temperaturen von 149 bis 371° C (300 bis 700° F), vorzugsweise bis 316° C (600° F) in Anwesenheit oder in Abwesenheit von Katalysatoren wie Licht, Eisen-(III)-chlorid, Antimon-(III)-chlorid, Cer-III-chlorid und Zinkchlorid hergestellt.

Bei der Anwendung dieser Verfahren zur Herstellung von Terephthalsäure- und Isophthalsäuredichlorid treten jedoch Schwierigkeiten auf, da sich bei der Chlorierung von Terephthalsäure- und Isophthalsäuredimethylester ein Sublimat bildet, das leicht zur Verstopfung der Abgasleitungen und infolgedessen zu einem gefährlichen Druckanstieg im Reaktionsgefäss führt.

In der DE-AS Nr. 1152400 wird vorgeschlagen, diese störende Sublimatbildung dadurch zu vermeiden, dass man die Chlorierung von Terephthalsäure- und Isophthalsäuredimethylester in Gegenwart von 2 bis 10 Gew.-% flüssiger Chloride und Methylester aromatischer Monocarbonsäuren, deren Siedepunkt im Bereich der Umsetzungstemperatur liegt, durchführt. Dieses Verfahren hat jedoch den Nachteil, dass die Aufarbeitung des Reaktionsgemisches durch die Anwesenheit der die Sublimatbildung verhindernden Monocarbonsäurechloride, die von dem zu isolierenden Terephthalsäure- und/oder Isophthalsäuredichlorid abgetrennt werden müssen, technisch sehr aufwendig wird. Terephthalsäure- und Isophthalsäuredichlorid dienen zu Herstellung von hochmolekularen Polykondensaten, z.B. aromatischen Polyamiden und aromatischen Polyestern; bei dieser Weiterverarbeitung stören bereits äusserst geringe Mengen an Monocarbonsäurechloriden, da diese als Kettenabbrecher wirken. Schon geringste Mengen an Verunreinigungen im Isophthalsäure- und Terephthalsäuredichlorid verursachen eine entscheidende Verringerung der Qualität der daraus hergestellten aromatischen Polyamide und Polyester. Deshalb werden an die Reinheit von Terephthalsäure- und Isophthalsäuredichlorid äusserst strenge Anforderungen gestellt.

Es wurde nun gefunden, dass sich die Nachteile der bekannten Verfahren zur Herstellung von Terephthalsäure- und Isophthalsäuredichlorid durch Chlorieren der entsprechenden Dimethylester dadurch vermeiden lassen, dass man dem Terephthalsäure- und/oder Isophthalsäuredimethylester für die Chlorierung 20 bis 200 Gew.-% bezogen auf das Gewicht des zu chlorierenden Phthalsäuredimethylesters an Terephthalsäure- und/oder Isophthalsäuredichlorid zusetzt. Durch diese Massnahme wird zum einen die störende Sublimatbildung verhindert und zum anderen ein Chlorierungsprodukt erhalten, aus dem sich Terephthalsäure- und Isophthalsäuredichlorid durch fraktionierte Destillation ohne besonderen technischen Aufwand in hohen Ausbeuten und in bislang unerreicht hoher Reinheit isolieren lassen.

Die Erfindung betrifft daher ein Verfahren zur Herstellung von Terephthalsäure- und Isophthalsäuredichlorid durch Chlorieren von Terephthalsäure- und Isophthalsäuredimethylester mit Chlorgas bei Temperaturen von 100 bis 250° C, gegebenenfalls in Gegenwart von Licht, und/oder die Seitenkettenchlorierung aromatischer Verbindungen fördernder Katalysatoren, das dadurch gekennzeichnet ist, dass man dem Terephthalsäure- und/oder Isophthalsäuredimethylester für die Chlorierung 20 bis 200 Gew.-% bezogen auf das Gewicht des zu chlorierenden Tere- und/oder Isophthalsäuredimethylesters Terephthalsäure- und/oder Isophthalsäuredichlorid zusetzt.

Terephthalsäure- und/oder Isophthalsäuredichlorid werden dem zu chlorierenden Terephthalsäure- und/oder Isophthalsäuredimethylester in einer Menge von 30 bis 100 Gew.-%, bezogen auf das Gewicht des zu chlorierenden Terephthalsäure- und/oder Isophthalsäuredimethylesters, zugesetzt.

Die Isolierung des Terephthalsäure- und/oder Isophthalsäuredichlorids aus dem nach dem erfindungsgemässen Verfahren erhaltenen Reaktionsgemisch erfolgt in üblicher Weise durch fraktionierte Destillation.

In Beispiel 1 der DE-AS Nr. 1152400 wird zwar die Rückführung eines Vorlaufs beschrieben, der neben Benzoylchlorid noch Terephthalsäuredichlorid enthält. Die Menge an Terephthalsäuredichlorid in diesem Vorlauf ist jedoch zu gering, um die störende Sublimatbildung zu verhindern.

In einer bevorzugten Ausführungsform des erfindungsgemässen Verfahrens wird die in Gegenwart von Terephthalsäure- und/oder Isophthalsäuredichlorid durchgeführte Chlorierung von Terephthalsäure- und Isophthalsäuredimethylester bereits abgebrochen, wenn der Gehalt an Terephthalsäure- und/oder Isophthalsäuredichlorid im Reaktionsgemisch erst auf 90 bis 98 Gew.-% angestiegen ist. Durch diese Massnahme wird eine Überchlorierung unter Bildung von Kernchlorierungs- und Chlorolyseprodukten vermieden. Diese unerwünschten Nebenprodukte lassen sich mit normalem Destillationsaufwand nur unvollkommen vom Terephthalsäure- und/oder Isophthalsäuredichlorid abtrennen und führen zu einer Beeinträchtigung der Qualität des Terephthalsäure- und Isophthalsäuredichlorids. Überraschenderweise wurde gefunden, dass die bei der unvollständigen Chlorierung noch im Reaktionsgemisch befindlichen Zwischenprodukte bei der Destillation des Reaktionsgemisches zum grossen Teil unter Bildung der gewünschten Dichloride zersetzen oder aber als partiell chlorierte Produkte im Nachlauf der Destillation zurückbleiben. Dieser Nachlauf wird, da er in der Regel auch grössere Mengen an Terephthalsäure- und/oder Isophthalsäuredichlorid enthält, im nächsten Chlorierungsansatz wieder eingesetzt. D.h. das erfindungsgemäss zu verwendende Terephthalsäure- und/oder Isophthalsäuredichlorid kann dem zu chlorierenden Terephthalsäure- und/oder Isophthalsäuredimethylester sowohl in Form der reinen Verbindungen bzw. von Gemischen der reinen Verbindungen als auch in Form dieser Chloride enthaltender Fraktionen aus der Terephthalsäure- und/oder Isophthalsäuredichlorid-Herstellung zugesetzt werden.

Die erfindungsgemässe Chlorierung von Terephthalsäure- und/oder Isophthalsäuredimethylester wird bei Temperaturen von 100 bis 250° C, vorzugsweise von 150 bis 200° C, ausgeführt. Die Chlorierung kann in Gegenwart von Licht, z.B. UV-strahlenreichem Licht, und/oder in Gegenwart von die Seitenkettenchlorierung aromatischer Verbindungen fördernden Radikalbildnern wie Azodiisobuttersäurenitril vorgenommen werden.

Das erfindungsgemässe Verfahren kann in Abwesenheit oder in Anwesenheit von unter den Reaktionsbedingungen inerten organischen Lösungsmitteln durchgeführt werden. Als inerte organische Lösungsmittel kommen z.B. halogenierte Kohlenwasserstoffe wie Hexachlorbutadien in Betracht. Die Chorierung kann sowohl diskontinuierlich als auch kontinuierlich, z.B. in einer Kaskade von 2 bis 10 Reaktoren, durchgeführt werden. Die Ausgangsverbindungen Terephthalsäure- und Isophthalsäuredimethylester können sowohl für sich allein als auch in Mischung eingesetzt werden. Gegebenenfalls kann das eingesetzte Chlorgas durch inerte Gase, wie z.B. Stickstoff, verdünnt sein.

Das erfindungsgemässe Verfahren weist gegenüber den bekannten Verfahren zur Herstellung von Terephthalsäure- und Isophthalsäuredichlorid wesentliche Vorteile auf: Die Säurechloride werden in hohen Ausbeuten und höheren Reinheitsgraden, als diese bislang erreichbar waren, erhalten, und die störende Sublimatbildung und mit ihr die Gefahr der Verstopfung der Abgasleitungen wird vermieden.

Terephthalsäure- und Isophthalsäuredichlorid sind wichtige Zwischenprodukte für die Herstellung von aromatischen Polyestern und Polyamiden.

*Beispiel 1*

Ein zylindrisches Chlorierungsgefäss, versehen mit Thermometer, Rückflusskühler, Gaseinleitungsrohr (Fritte) und einer in der Mitte des Gefässes angebrachten Lichtquelle (superaktinische 20-Watt-Leuchtstoffröhre) wird mit 777 g (4 Mol) Terephthalsäuredimethylester und 406 g Terephthalsäuredichlorid beschickt. Der Gefässinhalt wird auf 150° C erhitzt. Dann wird unter Bestrahlung mit Licht trockenes Chlorgas in den ersten 6 Stunden mit einer Geschwindigkeit von 50 l/h und anschliessend, entsprechend der verringerten Aufnahme, mit geringerer Geschwindigkeit eingeleitet. Insgesamt werden innerhalb von 10 Stunden 1310 g Chlor eingeleitet. Während der Chlorierung tritt keine störende Sublimatbildung auf.

Nach Beendigung der Chlorierung wird das Reaktionsgemisch durch Einblasen von Stickstoff von gelöstem Chlor und anderen gelösten Gasen befreit. Anschliessend wird die Reaktionsmischung unter verringertem Druck (12,0 bis 13,3 mbar) destilliert. Es werden 1194 g farbloses Terepthalsäuredichlorid erhalten; Siedepunkt 131-134° C; Schmelzpunkt 82° C; Reinheitsgrad 99,9 %.

Ausbeute: 97,0% der Theorie.

Nimmt man die Chlorierung in Abwesenheit von Terephthalsäuredichlorid unter sonst gleichen Bedingungen vor, so tritt eine starke Sublimatbildung während der Chlorierung auf.

Die entsprechende Chlorierung von Terephthalsäuredimethylester in Gegenwart von 5% Benzoylchlorid gemäss der DE-AS Nr. 1152400 anstelle des Zusatzes von Terephthalsäuredichlorid ergibt nach der Destillation als Hauptlauf Terephthalsäuredichlorid mit einem Reinheitsgrad von 99,7%. Unter den Verunreinigungen sind 0,13% Benzoylchlorid nachweisbar.

*Beispiel 2*

In der in Beispiel 1 beschriebenen Apparatur, in der lediglich die Lichtquelle fortgelassen wurde, werden 971 g (5 Mol) Isophthalsäuredimethylester und 406 g Isophthalsäuredichlorid vorgelegt. Bei 200° C wird trockenes Chlor zunächst mit einer Geschwindigkeit von 50 l/h, dann, nach 8 Stunden, entsprechend der verringerten Aufnahme, mit geringerer Geschwindigkeit eingeleitet. Insgesamt werden innerhalb von 13 Stunden 1710 g Chlor aufgenommen. Während der Chlorierung tritt keine störende Sublimatbildung auf.

Nach Beendigung der Chlorierung wird das Reaktionsgemisch durch Einblasen von Stickstoff von gelöstem Chlor und anderen gelösten Gasen befreit und anschliessend unter vermindertem Druck (12 bis 13,3 mbar) destilliert. Es werden 1385 g farbloses Isophthalsäuredichlorid erhalten. Siedepunkt 134-137° C; Schmelzpunkt 43° C; Reinheitsgrad 99,9%.

Ausbeute: 96,4% der Theorie.

*Beispiel 3*

In die in Beispiel 1 beschriebene Apparatur werden 388 g (2 Mol) Terephthalsäuredimethylester, 388 g (2 Mol) Isophthalsäuredimethylester, 203 g Terephthalsäure- und 203 g Isophthalsäuredichlorid eingefüllt. Bei 160° C werden unter Belichtung innerhalb von 9,5 Stunden insgesamt 1340 g trockenes Chlor eingeleitet. Während des Chlorierens tritt keine störende Sublimatbildung auf.

Nach Beendigung der Chlorierung wird das Reaktionsgemisch durch Einblasen von Stickstoff von gelöstem Chlor und anderen Gasen befreit.

Anschliessend wird das Reaktionsgemisch unter vermindertem Druck (12,0 bis 13,3 mbar) destilliert. Es werden 1193 g eines farblosen Gemisches aus Terephthalsäure- und Isophthalsäuredichlorid erhalten. Siedepunkt 132-137° C; Reinheitsgrad 99,9%.

Ausbeute: 96,9% der Theorie.

*Beispiel 4*

Die Chlorierung wird wie in Beispiel 1 beschrieben durchgeführt, nur wird die Chlorierung bei einem Gehalt an Terephthalsäuredichlorid von 94,4% im Chlorierungsansatz vorzeitig abgebrochen.

Nach Entfernen des im Reaktionsgemisch gelösten Chlors und anderer Gase durch Einleiten von Stickstoff wird das Reaktionsgemisch unter vermindertem Druck fraktioniert destilliert. Es werden 771 g farbloses Terephthalsäuredichlorid erhalten. Siedepunkt 140-141° C/21 mbar; Schmelzpunkt 82° C; Reinheitsgrad ≧ 99,9%. Als Nachlauf fallen 426 g eines Produktes an, das 85,4% Terephthalsäuredichlorid, 12,2% Terephthalsäurechlormethylesterchlorid, 0,7% Terephthalsäuredi-(chlormethyl)-ester und 1,7% unbekannte Verbindungen enthält.

Dieser Nachlauf wird im folgenden Ansatz mit 777 g (4 Mol) Terephthalsäuredimethylester vermischt und erneut der im Beispiel 1 beschriebenen Chlorierung unterworfen. Auch bei dieser Chlorierung wird keine Sublimatbildung beobachtet.

Bei der Destillation dieses zweiten Ansatzes werden als Hauptlauf 784 g Terephthalsäuredichlorid (Reinheitsgrad ≧ 99,9%) und, als Nachlauf, 431 g eines Terephthalsäuredichlorid-haltigen Nachlaufs erhalten.

Dieser Nachlauf wird erneut im nächsten Ansatz wiederverwendet.

## Patentansprüche

1. Verfahren zur Herstellung von Terephthalsäure- und Isophthalsäuredichlorid durch Chlorieren von Terephthalsäure- und Isophthalsäuredimethylester mit Chlorgas bei Temperaturen von 100 bis 250° C, gegebenenfalls in Gegenwart von Licht und/oder die Seitenkettenchlorierung aromatischer Verbindungen förderndern Katalysatoren, dadurch gekennzeichnet, dass man dem Terephthalsäure- und/oder Isophthalsäuredimethylester für die Chlorierung 30 bis 100 Gew.-%, bezogen auf das Gewicht des Terephthalsäure- und/oder Isophthalsäuredimethylesters, Terephthalsäure- und/oder Isophthalsäuredichlorid zusetzt.

2. Verfahren gemäss Anspruch 1, dadurch gekennzeichnet, dass man die Chlorierung abbricht, wenn der Gehalt an Terephthalsäure- und/oder Isophthalsäuredichlorid im Reaktionsgemisch auf 90 bis 98 Gew.-% des theoretisch möglichen Gehaltes angestiegen ist.

3. Verfahren gemäss Anspruch 2, dadurch gekennzeichnet, dass man das Chlorierungsgemisch fraktioniert destilliert und den Terephthalsäure-und/oder Isophthalsäuredichlorid-haltigen Destillationsnachlauf dem Terephthalsäure- und/oder Isophthalsäuredimethylester für die Chlorierung zusetzt.

## Claims

1. Process for the preparation of terephthaloyl dichloride and isophthaloyl dichloride by chlorination of dimethyl terephthalate and dimethyl isophthalate with chlorine gas at temperatures of 100 to 250° C, optionally in the presence of light and/or catalysts promoting side-chain chlorination of aromatic compounds, characterized in that 30 to 100% by weight, relative to the weight of the dimethyl terephthalate and/or dimethyl isophthalate, of terephthaloyl dichloride and/or isophthaloyl dichloride are added to the dimethyl terephthalate and/or dimethyl isophthalate for the chlorination.

2. Process according to Claim 1, characterized in that the chlorination is discontinued when the content of terephthaloyl dichloride and/or isophthaloyl dichloride in the reaction mixture has risen to 90 to 98% by weight of the theoretically possible content.

3. Process according to Claim 2, characterized in that the chlorination mixture is fractionally distilled and the tailings from the distillation which contain terephthaloyl dichloride and/or isophthaloyl dichloride are added to the dimethyl terephthalate and/or dimethyl isophthalate for the chlorination.

## Revendications

1. Procédé de préparation du dichlorure de l'acide téréphtalique et du dichlorure de l'acide isophtalique par chloration de téréphtalate de diméthyle et d'isophtalate de diméthyle par du chlore gazeux à des températures de 100 à 250° C, éventuellement en présence de la lumière et/ou de catalyseurs accélérant la chloration des chaînes latérales de composés aromatiques, procédé caractérisé en ce qu'on ajoute, au téréphtalate de diméthyle et/ou à l'isophtalate de diméthyle, pour la chloration, 30 à 100% en poids, par rapport au poids du téréphtalate de diméthyle et/ou de l'isophtalate de diméthyle, de dichlorure d'acide téréphtalique et/ou de dichlorure d'acide isophtalique.

2. Procédé selon la revendication 1, caractérisé en ce qu'on arrête la chloration quand la teneur en dichlorure de l'acide téréphtalique et/ou dichlorure de l'acide isophtalique dans le mélange réactionnel a augmenté pour atteindre 90 à 98% de la teneur théoriquement possible.

3. Procédé selon la revendication 2, caractérisé en ce qu'on soumet le mélange de chloration à une distillation fractionnée et l'on ajoute la queue de distillation, contenant du dichlorure d'acide téréphtalique et/ou du dichlorure d'acide isophtalique, au téréphtalate de diméthyle et/ou à l'isophtalate de diméthyle destiné à la chloration.